# FASCICULE DE BREVET EUROPEEN

(11) **EP 4 096 946 B1**
(45) Date de publication et mention de la délivrance du brevet: **17.04.2024**
(21) Numéro de dépôt: 21706641.4
(22) Date de dépôt: 28.01.2021
(51) Int. Cl.: B60H 3/02

(54) **SYSTEME DE NEBULISATION POUR VEHICULE AUTOMOBILE**
ZERSTÄUBERSYSTEM FÜR EIN KRAFTFAHRZEUG
NEBULISER SYSTEM FOR A MOTOR VEHICLE

(30) Priorité: 29.01.2020 FR 2000857
(43) Date de publication de la demande: 07.12.2022
(73) Titulaire: VALEO SYSTEMES THERMIQUES, 78320 Le Mesnil Saint-Denis (FR)
(72) Inventeur: FEUILLARD, Vincent, 78320 LE MESNIL-SAINT-DENIS (FR); MIKHEL, Julien, 78320 LE MESNIL SAINT-DENIS (FR); ESCULIER, Clément, 78320 LE MESNIL SAINT-DENIS (FR); ROUSSEAU, Yves, 78320 LE MESNIL-SAINT-DENIS (FR)
(74) Mandataire: Valeo Systèmes Thermiques
(86) Numéro de dépôt international: PCT/FR2021/050155
(87) Numéro de publication internationale: WO 2021/152260

(56) Documents cités:
- WO-A1-2019/048761
- CN-A- 108 163 382
- CN-A- 109 318 688
- FR-A1- 3 008 168
- FR-A1- 3 043 920

## Description

L'invention a pour objet un système de nébulisation pour véhicule automobile.

De façon connue, un système de nébulisation comprend un réservoir pour stocker un liquide d'eau liquide et un dispositif d'émission d'ondes acoustiques configuré de sorte que l'eau issue du réservoir pour stocker un liquide forme un brouillard de gouttelettes d'eau.

Un tel système de nébulisation permet de rafraîchir un flux d'air dans lequel le brouillard d'eau est pulvérisé.

Dans un véhicule automobile, même muni d'un dispositif de ventilation et/ou climatisation, il n'est pas rare que les usagers du véhicule souffrent de la chaleur dans l'habitacle, en particulier les passagers arrière, plus éloignés des aérateurs du dispositif de ventilation et/ou climatisation.

De ce fait, il est intéressant de recourir à un système de nébulisation, puisqu'alors le brouillard d'eau rafraîchit rapidement l'air de l'habitacle, assurant une sensation de froid immédiat tout en le ré-humidifiant.

Du fait que l'eau issue du système de nébulisation est en contact direct avec la peau des utilisateurs du véhicule, il est primordial de s'assurer que le système de nébulisation est sain. Le document FR 3 008 168 A1 divulgue un système de nébulisation connu comprenant un dispositif électroluminescent configuré pour émettre un rayonnement dans le spectre ultraviolet.

D'autre part, le système de nébulisation, parce qu'il constitue un élément supplémentaire du véhicule qu'il occupe, doit être le moins encombrant possible.

Le but de l'invention est de proposer un système de nébulisation sans danger pour la santé des usagers du véhicule automobile tout en étant compact.

A cet effet, l'invention a pour objet un système de nébulisation pour véhicule automobile, comprenant au moins un réservoir pour stocker un liquide, une enceinte de nébulisation, ladite enceinte de nébulisation comprenant un moyen de nébulisation muni d'un dispositif d'émission d'ondes acoustiques configuré de sorte que ledit liquide forme un brouillard de gouttelettes dudit liquide, le brouillard étant destiné à pénétrer dans un habitacle du véhicule automobile, un dispositif électroluminescent configuré pour émettre un rayonnement dans le spectre ultraviolet, et un support dudit dispositif électroluminescent, ledit au moins un réservoir pour stocker un liquide étant conformé pour être traversé par le support du dispositif électroluminescent.

Ainsi, grâce au dispositif électroluminescent, les bactéries pouvant proliférer dans le liquide circulant dans système de nébulisation sont efficacement neutralisées, sans qu'il soit nécessaire de recourir à un filtre, la forme même du support assurant une plus grande compacité du système de nébulisation.

Selon une autre caractéristique de l'invention, le spectre ultraviolet comprend des longueurs d'onde dans un intervalle entre 200 nm et 280 nm.

Selon une autre caractéristique de l'invention, ledit au moins un réservoir pour stocker un liquide comprend un orifice de passage du dispositif électroluminescent.

Selon une autre caractéristique de l'invention, ledit au moins un réservoir pour stocker un liquide comprend un puits traversant formant logement du support du dispositif électroluminescent.

Selon une autre caractéristique de l'invention, le support du dispositif électroluminescent comprend un conduit conformé pour être disposé dans le logement dudit au moins un réservoir pour stocker un liquide.

Selon une autre caractéristique de l'invention, le conduit délimite un espace interne creux de sorte à assurer une alimentation électrique du dispositif électroluminescent.

Selon une autre caractéristique de l'invention, ledit au moins un réservoir pour stocker un liquide comprend une paroi sur laquelle repose ledit dispositif électroluminescent.

Selon une autre caractéristique de l'invention, le dispositif électroluminescent est orienté de sorte à irradier le brouillard de gouttelettes dans l'enceinte de nébulisation.

Selon une autre caractéristique de l'invention, le système comprend un dissipateur thermique associé au dispositif électroluminescent.

Selon une autre caractéristique de l'invention, le dissipateur comprend une cavité de logement du support du dispositif électroluminescent.

L'invention a également pour objet un dispositif de ventilation, chauffage et/ou climatisation pour véhicule automobile, comprenant un système de nébulisation tel que précédemment décrit.

### Brève description des dessins

D'autres caractéristiques, détails et avantages de l'invention apparaîtront à la lecture de la description détaillée ci-après, et à l'analyse des dessins annexés, sur lesquels :
**Fig. 1**

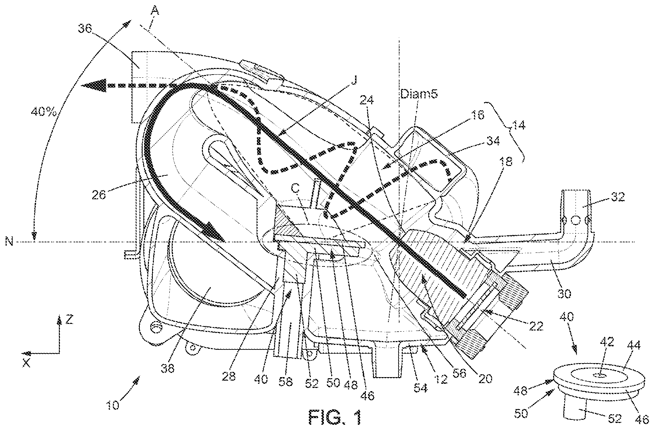

   [Fig. 1] illustre une vue partielle de face d'un système de nébulisation selon la présente invention.
**Fig. 2**

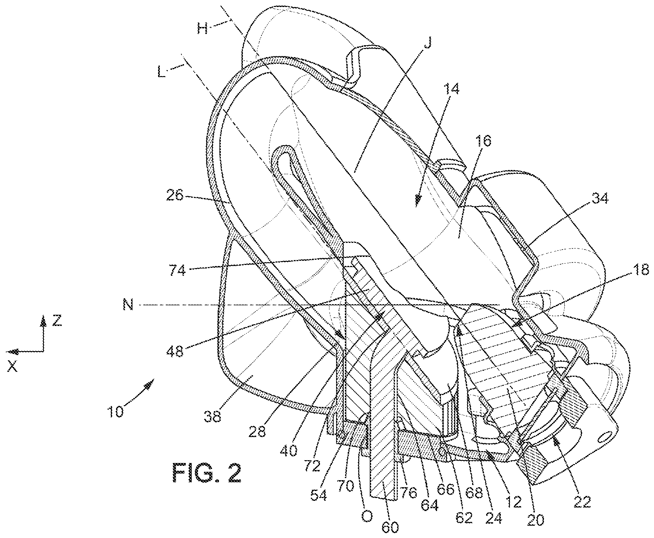

   [Fig. 2] illustre une vue en perspective partielle et en coupe du système de la figure 1 selon un autre mode de réalisation.

### Description des modes de réalisation

### Nébuliseur

L'invention a pour objet un système de nébulisation pour un véhicule automobile, référencé 1 sur la figure, encore appelé nébuliseur 10.

Le nébuliseur 10 permet de rafraîchir un flux d'air à destination de l'habitacle du véhicule automobile, comme il va être détaillé. Le nébuliseur 10 est destiné à être intégré dans une console centrale du véhicule, notamment.

Le nébuliseur est décrit selon la configuration spécifique des figures 1 et 2 mais il est bien entendu que l'invention ne se limite pas à ces modes de réalisation.

Comme visible sur les figures, le nébuliseur 10 comprend un réservoir de stockage d'un fluide, 12, préférentiellement de l'eau liquide, détaillé ultérieurement, autrement appelé cuve.

Le nébuliseur 10 comprend également une enceinte de nébulisation 14. L'enceinte de nébulisation 14 est réalisée ici sous la forme d'un corps creux allongé s'étendant selon un axe longitudinal A.

Dans le mode de réalisation illustré aux figures, l'axe longitudinal A est incliné par rapport à une direction verticale Z et par rapport à une direction horizontale X.

Comme il ressort également des figures, l'enceinte de nébulisation 14 comporte au moins deux parties disposées, l'une à la suite de l'autre selon la direction de l'axe longitudinal A, à savoir une chambre de nébulisation 16 et une cheminée de nébulisation 18, la cheminée de nébulisation 18 débouchant dans la chambre de nébulisation 14.

Comme il ressort des figures 1 et 2, la cheminée de nébulisation 18 comporte un moyen de nébulisation réalisé ici sous la forme d'une buse de nébulisation 20 munie d'un dispositif d'émission d'ondes acoustiques transmises dans le liquide configuré de sorte que la surface du liquide issu du réservoir pour stocker le liquide 12 génère un brouillard de gouttelettes du liquide, ce brouillard étant destiné à pénétrer dans un habitacle du véhicule automobile.

On note que l'axe longitudinal A correspond également à la direction avec laquelle le jet J d'eau dont se détache le brouillard est éjecté de la buse de nébulisation 20 dans la chambre de nébulisation 16.

Comme visible sur les figures, l'axe longitudinal de la buse de nébulisation 22 coïncide avec l'axe longitudinal A de l'enceinte de nébulisation 14.

La buse de nébulisation 20 est disposée au moins en partie à l'intérieur de l'enceinte de nébulisation 14. La buse de nébulisation 20 comporte une paroi latérale délimitant un volume intérieur apte à contenir le liquide à nébuliser. La section transversale intérieure de cette paroi latérale présente un rétrécissement progressif en direction d'un orifice de sortie pour le liquide 24 permettant de former un concentrateur d'ondes acoustiques.

Un élément (céramique) piézo-électrique 22 est disposé à l'opposé d'un l'orifice de sortie 24 pour le liquide.

L'élément piézo-électrique est apte à émettre des ondes acoustiques dans le liquide à pulvériser ce qui permet de générer un brouillard de gouttelettes de liquide lorsque la buse de nébulisation 20 est remplie par ce dernier et lorsque l'élément piézo-électrique émet des ondes acoustiques de fréquence et d'intensité appropriées. De manière préférentielle, l'élément piézo-électrique pourra émettre des ultrasons dont la fréquence sera comprise entre 1 MHz et 3 MHz, notamment entre 1,7 MHz et 2,4 MHz.

Par exemple, le diamètre des gouttelettes contenues dans le brouillard est inférieur à 10 µm.

La buse de nébulisation 20 comporte aussi au moins un orifice d'admission pour le liquide à nébuliser qui permet l'introduction du liquide à nébuliser dans le volume intérieur de la buse, en communication fluidique avec le réservoir pour stocker le liquide 12.

Comme il ressort des figures 1 et 2, le système de nébulisation 10 comprend également un conduit 26 pour le retour du liquide dans le réservoir pour stocker le liquide 12. Le conduit 26 pour le retour du liquide prolonge la chambre de nébulisation 20 en formant un coude quasiment à 180° jusqu'à une extrémité 28. La forme coudée du conduit 26 pour le retour du liquide assure une compacité améliorée du système de nébulisation 10.

Le système de nébulisation 10 comprend également un canal 30 d'entrée d'eau muni d'un embout 32 destiné à recevoir une cartouche contenant du liquide, et constituant de ce fait un réservoir pour stocker le liquide supplémentaire, avantageusement amovible, du système 10, comme illustré sur la figure 1.

Le nébuliseur 10 comporte en outre un conduit d'entrée d'air 34 ainsi qu'au moins un conduit de sortie du brouillard 36, un ventilateur 38 forçant le mouvement de l'air.

Ainsi, l'air pénètre dans l'enceinte de nébulisation 14 au niveau de la chambre de nébulisation 16 où se produit le mélange entre l'air et le liquide nébulisé avant de quitter le système 10 par le conduit de sortie 36 où il circule jusqu'à l'habitacle du véhicule.

Le nébuliseur 10 comprend également un dispositif électroluminescent 40, illustré sur les figures 1 et 2.

Le dispositif électroluminescent 40 comporte une source de rayonnement ultraviolet 42, dont les longueurs d'onde émises sont entre 200 nm et 280 nm (il s'agit d'un rayonnement ultraviolet C). La part de rayonnement efficace, c'est-à-dire la part de rayonnement qui permet de tuer les bactéries est située entre 200 et 248 nm. Toutefois, la source de rayonnement peut émettre entre 248 et 400 nm même si les performances sont moindres en termes de proportion de bactéries tuées.

De manière très avantageuse, le dispositif électroluminescent 40 consiste en un réacteur à photolyse à diodes électroluminescentes émettant entre entre 200 et 280 nm. Les diodes électroluminescentes sont des sources de rayonnement ultraviolet présentant de nombreux avantages. Elles ont une faible consommation, fonctionnent en tension d'alimentation continue et n'engendrent pas de problème de compatibilité électromagnétique, ce qui facilite leur intégration au sein du système. Elles présentent également de faibles dimensions ce qui permet de conserver un système compact. De plus, elles ne nécessitent pas l'utilisation de mercure et présentent une longue durée de vie, ce qui représente un atout pour des problématiques de recyclage et de maintenance.

La source de rayonnement ultraviolet 42, ainsi qu'un circuit électronique associé, sont noyés dans une résine 44 bordée par un joint 46. Le dispositif électroluminescent 40 comprend un support 48, constitué par un socle 50, préférentiellement en inox, disposé sous la résine 44 et, avantageusement, un conduit 52, par exemple perpendiculaire au socle 50.

Comme il ressort des figures, le réservoir pour stocker le liquide 12 (autrement appelée cuve 12) est conformé pour être traversé par le support 48.

On décrit maintenant en détail le premier mode de réalisation, illustré sur la figure 1.

Sur la figure 1, la cuve 12 comprend une paroi de fond 54, plane, et une paroi supérieure, 56, opposée à la paroi de fond 30. Le dispositif électroluminescent 40 est disposé sur la paroi supérieure 32 de sorte que, en fonctionnement, le socle 50 est immergé dans l'eau tandis que la résine 44 est disposée au-dessus du niveau d'eau. L'immersion du socle 50 assure un refroidissement efficace du dispositif électroluminescent 40.

La cuve 12 comprend un puits 58 de logement du canal 52 du dispositif électroluminescent 40 traversant la cuve 12 entre deux extrémités creuses, l'une dans la paroi de fond, l'autre dans la paroi supérieure. Le puits 58 assure une étanchéité afin notamment d'alimenter électriquement le dispositif électroluminescent 40 en toute sécurité. Le puits traversant 58 assure également une plus grande compacité du système de nébulisation 10.

Sur la figure 1, le socle 50 est disposé parallèlement au niveau d'eau N dans la cuve 12, la source de rayonnement ultraviolet 42 étant orientée vers la chambre de nébulisation 20. Ainsi, le rayonnement émis par la source de rayonnement ultraviolet 42 délimite un cône C qui irradie le jet de brouillard s'écoulant dans la chambre de nébulisation 20. Cette configuration présente l'avantage de traiter directement l'air, où les bactéries prolifèrent particulièrement.

On note qu'en état de fonctionnement, on solidarise la cartouche contenant du liquide à l'embout dédié, et le niveau d'eau N atteint la conduite, la cheminée de nébulisation 18, l'extrémité du conduit retour, et la paroi supérieure, le puits 58 étant entouré d'eau.

On note également que cette configuration permet de ne pas équiper le système 10 de pompe de circulation d'eau.

On décrit maintenant en détail le deuxième mode de réalisation, illustré sur la figure 2.

Sur la figure 2, la cuve 12 comprend une paroi de fond 54, plane, comprenant un orifice O de passage du canal 60, comme il va être détaillé.

Comme également visible sur la figure 2, le système de nébulisation 10 comprend un dissipateur thermique 62 disposé entre la conduite retour 26 et la buse de nébulisation 20. Le dissipateur 62 est percé d'une cavité 64 de logement du support 52 du dispositif électroluminescent 40, traversant entre un orifice 70 dans une première paroi, dite basse, 66, et un autre orifice 72 dans une deuxième paroi, dite haute, 68.

La paroi basse 66 repose sur la paroi de fond 54 de la cuve 12, tandis que la paroi haute 68 fait face à une portion 74 de paroi de la chambre de nébulisation 16.

Comme illustré sur la figure 2, l'orifice 70 dans la paroi basse est disposé en regard de l'orifice O de la paroi de fond 54, un presse étoupe 76 assurant une étanchéité entre l'orifice de la paroi de fond O et l'orifice 70 de la paroi basse 66.

La paroi haute s'étend le long d'une direction L inclinée relativement aux axes X et Z, par exemple de 40° par rapport à l'axe X. Avantageusement, cette inclinaison correspond à la direction de la portion 74, ainsi qu'à la direction A d'éjection du brouillard J.

Cette configuration parallèle de la direction L à l'axe A permet à la chambre de nébulisation 16 de présenter une section plus circulaire que le premier mode de réalisation, ce qui améliore le tourbillonnement de l'air dans la chambre de nébulisation 16 et optimise l'entrainement du brouillard vers l'habitacle du véhicule.

Du fait de cette configuration parallèle, le dispositif électroluminescent 40 est disposé plus haut que pour le premier mode de réalisation, et se trouve au moins partiellement au-dessus du niveau d'eau N.

Sur la figure 2, la source de rayonnement ultraviolet 42 est orientée vers la chambre de nébulisation 20. Ainsi, le rayonnement émis par la source de rayonnement ultraviolet 42 délimite un cône qui irradie le jet de brouillard s'écoulant dans la chambre de nébulisation 20. Cette configuration présente l'avantage de traiter directement l'air, où les bactéries prolifèrent particulièrement.

On note qu'en état de fonctionnement, on solidarise la cartouche contenant du liquide à l'embout dédié, et le niveau d'eau N atteint la conduite, la cheminée de nébulisation 18, l'extrémité du conduit retour, et la paroi supérieure, le dissipateur 62 thermique étant immergé dans l'eau.

Si le support 50 n'est pas immergé, le dissipateur thermique assure un refroidissement suffisant du dispositif électroluminescent 40, notamment du fait de son immersion.

On note également que cette configuration permet de ne pas équiper le système 10 de pompe de circulation d'eau.

### Avantages apportés

Grâce au dispositif électroluminescent, les bactéries ne peuvent pas proliférer dans le nébuliseur 10, ce qui assure que le nébuliseur soit toujours sain, que le nébuliseur 10 soit en fonctionnement ou à l'arrêt.

## Revendications

1. Système de nébulisation pour véhicule automobile, comprenant :
- au moins un réservoir pour stocker un liquide (12),
- une enceinte de nébulisation (14), ladite enceinte de nébulisation (14) comprenant un moyen de nébulisation comprenant un dispositif d'émission d'ondes acoustiques (22) configuré de sorte que ledit liquide issu du réservoir pour stocker un liquide (12) forme un brouillard de gouttelettes dudit liquide, le brouillard étant destiné à pénétrer dans un habitacle du véhicule automobile,
- un dispositif électroluminescent (40) configuré pour émettre un rayonnement dans le spectre ultraviolet, et
- un support (50) dudit dispositif électroluminescent (40), ledit système étant **caractérisé en ce que** au moins un réservoir pour stocker un liquide (12) est conformé pour être traversé par le support (48) du dispositif électroluminescent (40).

2. Système selon la revendication 1, dans lequel le spectre ultraviolet comprend des longueurs d'onde dans un intervalle entre 200 nm et 280 nm.

3. Système selon l'une des revendications précédentes, dans lequel ledit au moins un réservoir pour stocker un liquide (12) comprend un orifice (O) de passage du dispositif électroluminescent (40).

4. Système selon l'une des revendications précédentes, dans lequel ledit au moins un réservoir pour stocker un liquide (12) comprend un puits traversant (58) formant logement du support (48) du dispositif électroluminescent (40).

5. Système selon l'une des revendications précédentes, dans lequel ledit au moins un réservoir pour stocker un liquide (12) comprend une paroi sur laquelle repose ledit dispositif électroluminescent (40).

6. Système selon l'une des revendications précédentes, dans lequel le dispositif électroluminescent (40) est orienté de sorte à irradier le brouillard de gouttelettes dans l'enceinte de nébulisation (14).

7. Système selon l'une des revendications précédentes, comprenant un dissipateur thermique (62) associé au dispositif électroluminescent (40).

8. Système selon l'une des revendications précédentes, dans lequel le dissipateur thermique (62) comprend une cavité (64) de logement du support (50) du dispositif électroluminescent (40).

9. Dispositif de ventilation, chauffage et/ou climatisation pour véhicule automobile, comprenant un système de nébulisation (10) selon l'une quelconque des revendications précédentes.

## Patentansprüche

1. Vernebelungssystem für ein Kraftfahrzeug, umfassend:
- mindestens einen Behälter zum Lagern einer Flüssigkeit (12),
- eine Vernebelungskammer (14), wobei die Vernebelungskammer (14) ein Vernebelungsmittel umfasst, das eine Vorrichtung zum Emittieren von Schallwellen (22) umfasst, die so ausgestaltet ist, dass die aus dem Behälter zum Lagern einer Flüssigkeit (12) stammende Flüssigkeit einen Tröpfchennebel der Flüssigkeit bildet, wobei der Nebel dazu bestimmt ist, in einen Innenraum des Kraftfahrzeugs einzudringen,
- eine lichtemittierende Vorrichtung (40), die dazu ausgestaltet ist, eine Strahlung im ultravioletten Spektrum zu emittieren, und
- einen Halter (50) der lichtemittierenden Vorrichtung (40),
wobei das System **dadurch gekennzeichnet ist, dass** mindestens ein Behälter zum Lagern einer Flüssigkeit (12) dazu ausgestaltet ist, von dem Halter (48) der lichtemittierenden Vorrichtung (40) durchquert zu werden.

2. System nach Anspruch 1, bei dem das ultraviolette Spektrum Wellenlängen in einem Intervall zwischen 200 nm und 280 nm umfasst.

3. System nach einem der vorhergehenden Ansprüche, bei dem der mindestens eine Behälter zum Lagern einer Flüssigkeit (12) eine Öffnung (0) zum Durchführen der lichtemittierenden Vorrichtung (40) umfasst.

4. System nach einem der vorhergehenden Ansprüche, bei dem der mindestens eine Behälter zum Lagern einer Flüssigkeit (12) einen durchgehenden Schacht (58) umfasst, der die Aufnahme des Halters (48) der lichtemittierenden Vorrichtung (40) bildet.

5. System nach einem der vorhergehenden Ansprüche, bei dem der mindestens eine Behälter zum Lagern einer Flüssigkeit (12) eine Wand umfasst, auf der die lichtemittierende Vorrichtung (40) aufliegt.

6. System nach einem der vorhergehenden Ansprüche, bei dem die lichtemittierende Vorrichtung (40) so ausgerichtet ist, dass sie den Tröpfchennebel in der Vernebelungskammer (14) bestrahlt.

7. System nach einem der vorhergehenden Ansprüche, das einen Wärmeableiter (62) umfasst, welcher der lichtemittierenden Vorrichtung (40) zugeordnet ist.

8. System nach einem der vorhergehenden Ansprüche, bei dem der Wärmeableiter (62) einen Hohlraum (64) zur Aufnahme des Halters (50) der lichtemittierenden Vorrichtung (40) umfasst.

9. Lüftungs-, Heizungs- und/oder Klimatisierungsvorrichtung für ein Kraftfahrzeug mit einem Vernebelungssystem (10) nach einem der vorhergehenden Ansprüche.

## Claims

1. Nebulizer system for a motor vehicle, comprising:
- at least one reservoir (12) for storing a liquid,
- a nebulizer enclosure (14), said nebulizer enclosure (14) comprising a nebulizing means comprising a device (22) for emitting acoustic waves that is configured such that said liquid coming from the reservoir (12) for storing a liquid forms a mist of droplets of said liquid, the mist being intended to enter an interior of the motor vehicle,
- an electroluminescent device (40) configured to emit radiation in the ultraviolet spectrum, and
- a support (50) for said electroluminescent device (40), said system being **characterized in that** at least one reservoir (12) for storing a liquid is configured so as to be traversed by the support (48) of the electroluminescent device (40).

2. System according to Claim 1, wherein the ultraviolet spectrum comprises wavelengths in a range of between 200 nm and 280 nm.

3. System according to either of the preceding claims, wherein said at least one reservoir (12) for storing a liquid comprises a through-orifice (0) for the electroluminescent device (40).

4. System according to one of the preceding claims, wherein said at least one reservoir (12) for storing a liquid comprises a through-well (58) forming a housing for the support (48) of the electroluminescent device (40) .

5. System according to one of the preceding clai ms, wherein said at least one reservoir (12) for storing a liquid comprises a wall on which said electroluminescent device (40) rests.

6. System according to one of the preceding claims, wherein the electroluminescent device (40) is oriented so as to irradiate the mist of droplets in the nebulizer enclosure (14).

7. System according to one of the preceding claims, comprising a heat sink (62) associated with the electroluminescent device (40).

8. System according to one of the preceding claims, wherein the heat sink (62) comprises a cavity (64) for housing the support (50) of the electroluminescent device (40) .

9. Ventilation, heating and/or air-conditioning device for a motor vehicle, comprising a nebulizer system (10) according to any one of the preceding claims.
